# EUROPEAN PATENT APPLICATION

(11) **EP 2 060 252 A1**
(43) Date of publication of application: **20.05.2009**
(21) Application number: 07291378.3
(22) Date of filing: 19.11.2007
(51) Int. Cl.: A61K 9/127, A61K 9/107, A61K 47/28, A61K 47/18, A61K 31/7032, A61K 31/7028, A61K 39/39

(54) **New formulation of galactosylceramide derivatives**

(71) Applicant: Wittycell, 51688 Reims Cédex 2 (FR)
(72) Inventor: Serra, Vincent, 91070 Bondoufle (FR)
(74) Representative: Colombet, Alain André

(57) **Abstract**

The present invention relates to a composition comprising at least one derivative of galactosylceramides, and at least one bile salt, such as sodium taurocholate or sodium deoxycholate.
It also relates to a method for the preparation of said composition, comprising the co-solubilization of the derivative of galactosylceramides and the bile salt into a solvent, followed by the evaporation of said solvent.

## Description

The present invention concerns a new formulation of galactosylceramide derivatives.

Glycolipids, such as galactosylceramides or derivatives thereof, need to be formulated in an optimal way in order to guarantee the reproducibility of their pharmacological effect as well as the scalability and robustness of the manufacturing process used to produce the final products in which they will be incorporated.

These glycolipids are considered as very low solubility compounds.

Preliminary work performed on a compound such as α-galactosylceramide (α-GC) has allowed defining an analytical method that could be adapted to other glycolipids of the series for preliminary characterization work. It was possible to use UV detection (205 nm), which appears to be a suitable option for further analytical development work. Solubility of this compound was found to be very poor in usual chromatographic solvents such as acetonitrile.

Thermodynamic aqueous solubility was also shown to be very poor (below 5 µg/mL) for α-GC.

The known method for preparing glycolipids formulations, such as galactosylceramides formulations, requires firstly dissolving glycolipids in a water-miscible organic solvent, followed by dispersing the obtained solution in an aqueous buffer, followed by sonication, sterilizing filtration and freeze-drying. These successive operations are implemented because of the likely marked tendency of the glycolipid suspension to agglomerate and to sediment.

Several attempts were made to improve the solubility of said galactosylceramides derivatives. Solubility was tested in various solutions containing injectable surfactants (such as Cremophor EL from BASF, Solutol HS15 from BASF, Tween80 from MERCK, and Pluronic F68 from BASF) or hydroxypropyl β-cyclodextrine (HPBCD from SIGMA) but such formulations did not show any solubility enhancement. Further, said galactosylceramide derivatives were sonicated in order to produce liposomes but such liposome preparations were prepared in presence of a charged phospholipid (sodium phosphatidyl glycerol) known to be able to enhance liposome suspensions colloidal stability, and none of these preparations led to any stable liposomal suspension.

The aim of the present invention is to provide alternative ways of formulating galactosylceramides or derivatives thereof, in order to improve the solubility of said galactosylceramides or derivatives thereof.

The present invention relates to a composition comprising:
- at least one derivative of galactosylceramides, and
- at least one bile salt.

The expression "derivative of galactosylceramides" designates galactosylceramides, such as α-galactosylceramide, and also derivatives thereof.

Derivatives of galactosylceramides are in particular 6"-amino-6"-deoxygalactosylceramide compounds as described in WO2004/094444, or bacterial glycolipids as described in WO2006/083671.

Derivatives of galactosylceramides also include the compound as described in WO2006/029010 of formula:

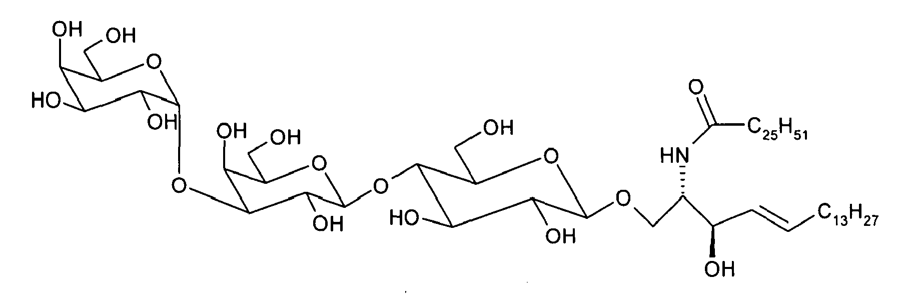

Other derivatives of galactosylceramides to be used in the composition of the present invention are those having the following formula: 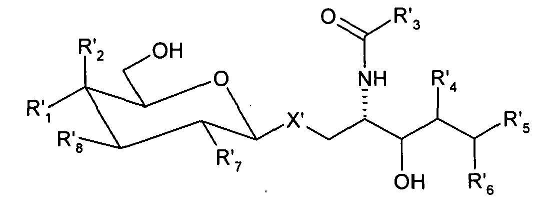 wherein:
X' is -O-, -CH₂- or -S-;
R'₁ is H, OH, OSO₃H, SO₃H, PO₄, PO₄H, COOH or a group having the following formula: 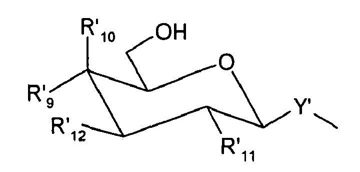 wherein:
   Y' is O, CH₂ or S;
   R'₉, R'₁₀, R'₁₁ and R'₁₂ are independently selected from: H, OH,
   OSO₃H, SO₃H, PO₄, PO₃H, and COOH;
R'₂ is selected from: H, OSO₃H, SO₃H, and PO₄;
R'₇ and R'₈ are independently selected from H, OH, OSO₃H, SO₃H, PO₄, PO₄H, and COOH;
R'₃ is a saturated or unsaturated hydrocarbon group having about 7 to about 25 carbon atoms;
R'₄ is H, OH, or, together with R'₆, forms a carbon-carbon double bond;
R'₅ is a saturated or unsaturated hydrocarbon group having about 5 to about 15 carbon atoms; and
R'₆ is H, OH, or, together with R'₄, forms a carbon-carbon double bond,
provided that at least one of R'₁, R'₂, R'₇, R'₈, R'₉, R'₁₀, R'₁₁ or R'₁₂ is H, OH, OSO₃H, SO₃H, PO₄, PO₄H, or COOH.

Other derivatives of galactosylceramides are described in WO03/009812 or WO 2005/102049 or WO 03/105769.

The composition of the present invention can also comprise derivatives of galactosylceramides as described in US 5,936,076 or US 5,780,441 or WO98/29534 or WO98/44928 or US 6,555,372.

The following compounds are also derivatives of galactosylceramides according to the present invention:

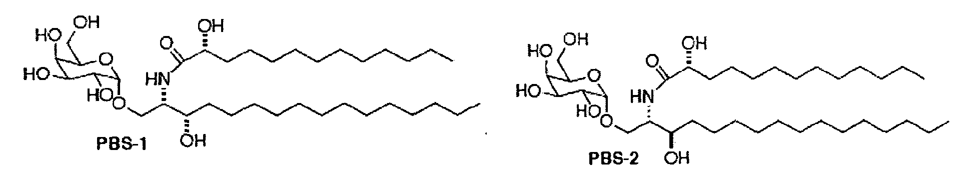

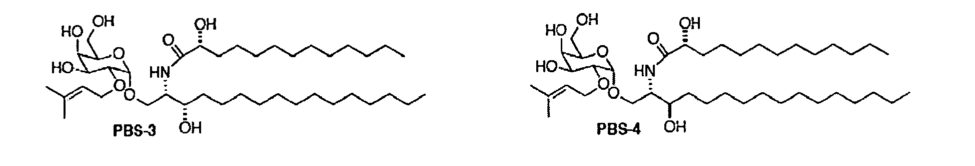

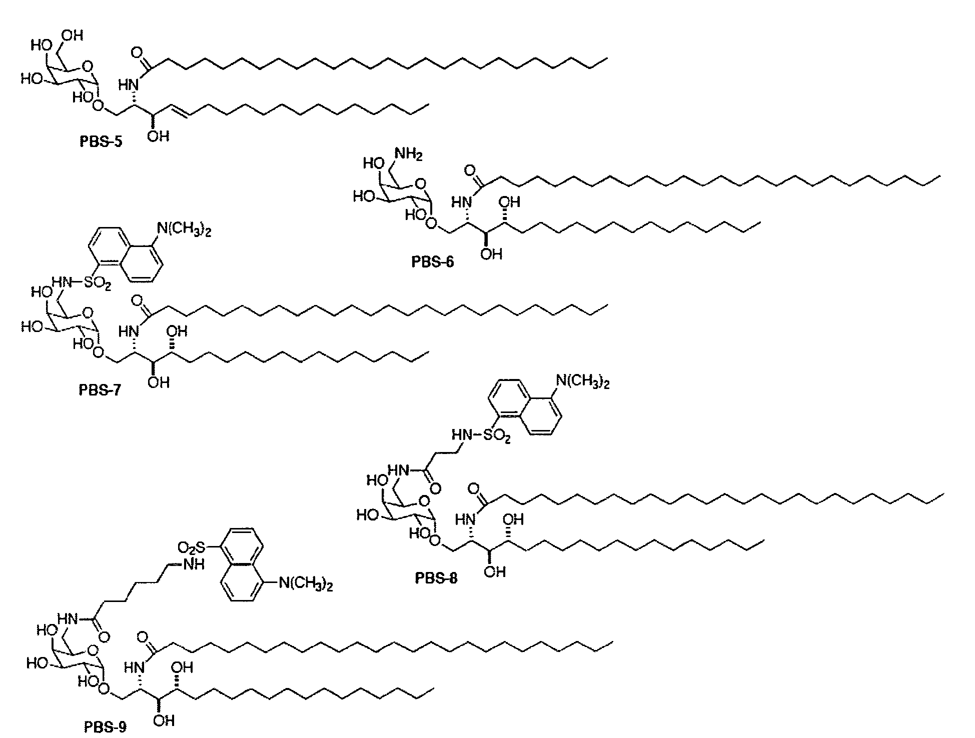

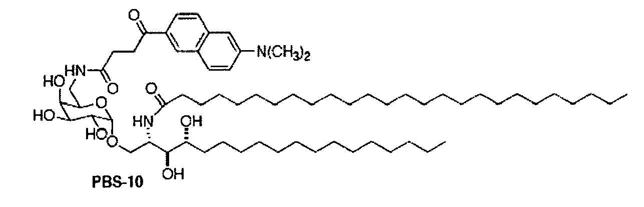

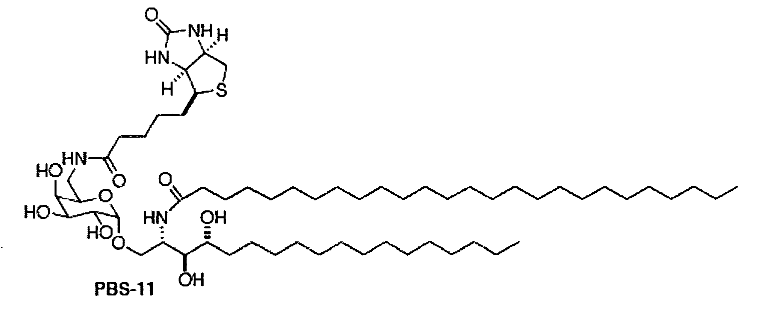

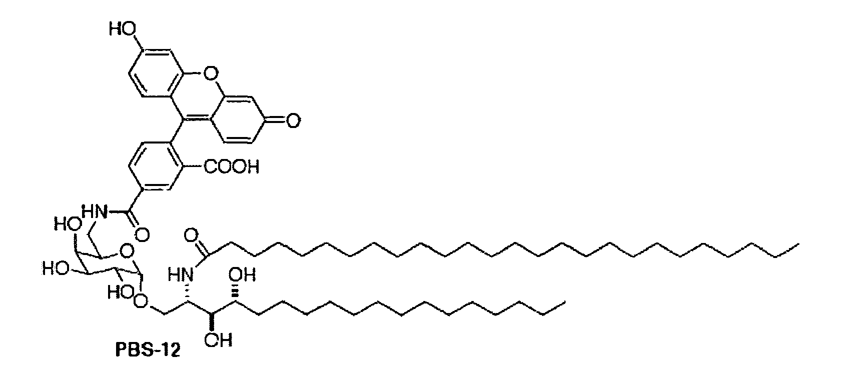

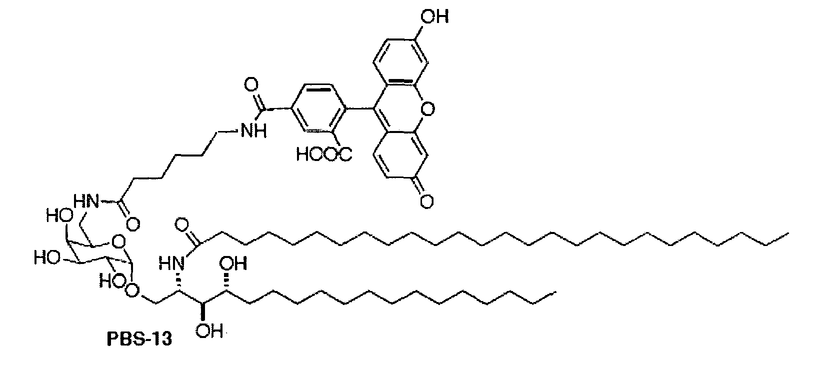

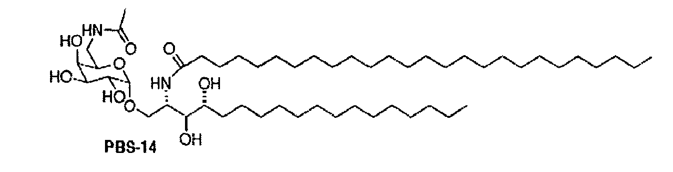

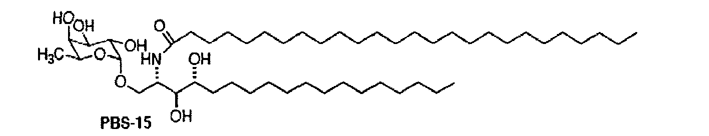

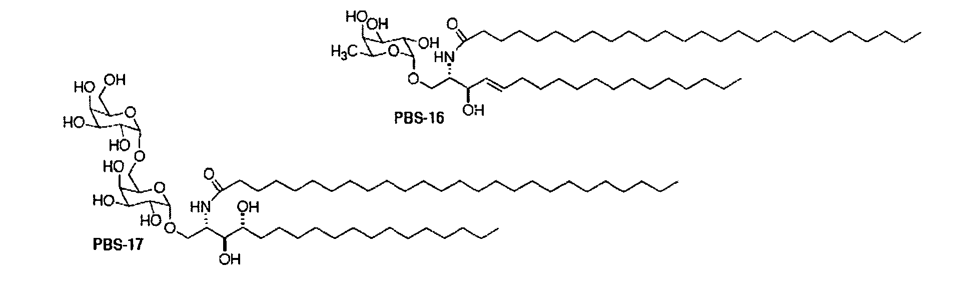

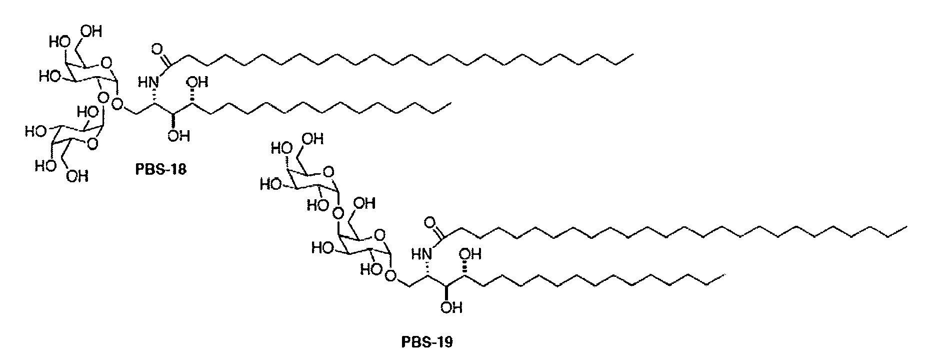

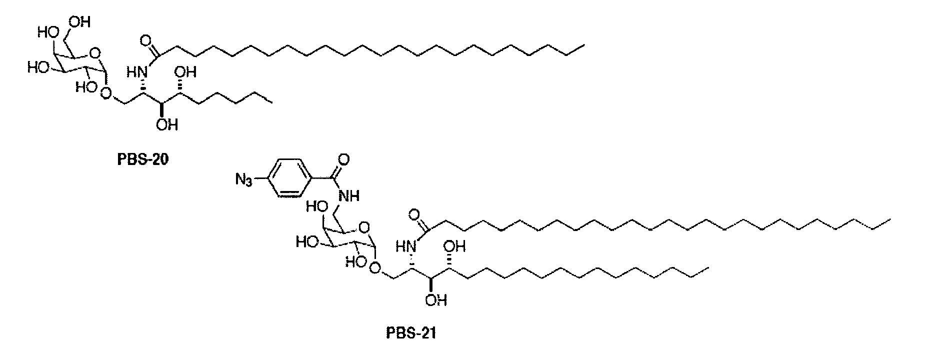

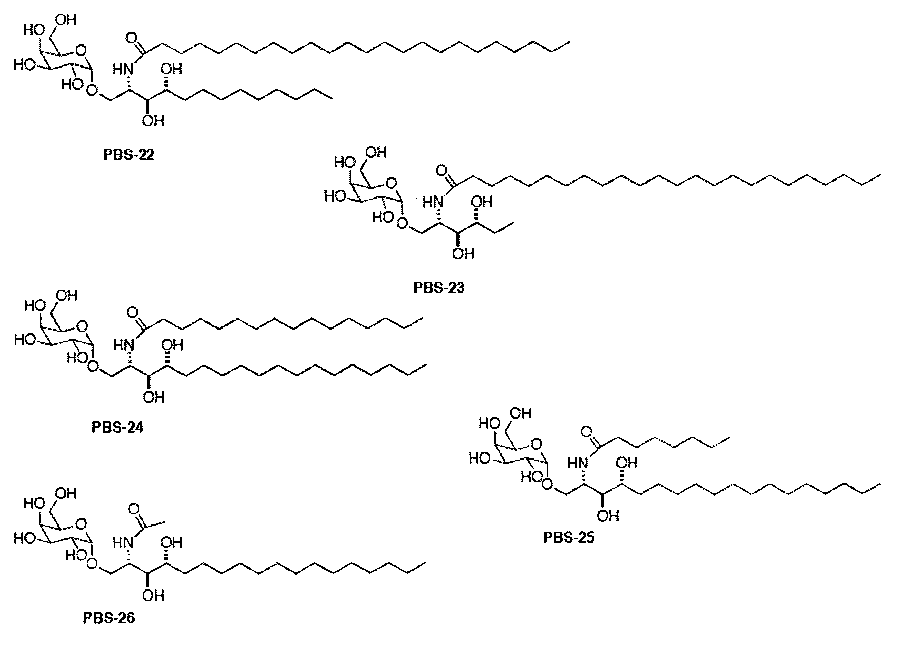

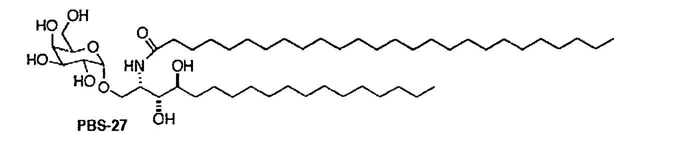

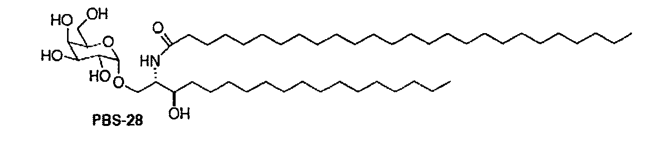

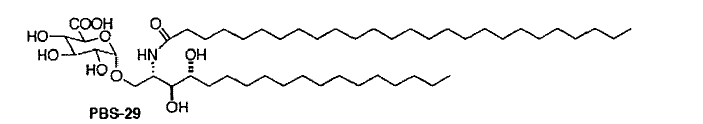

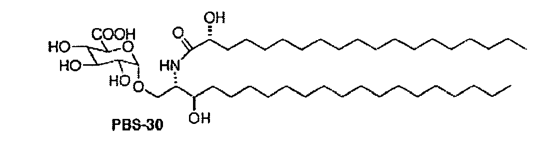

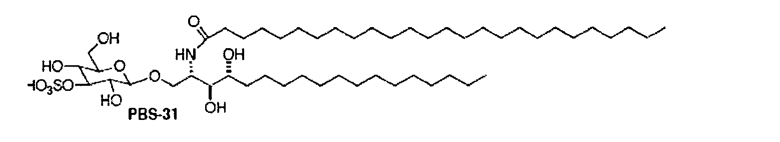

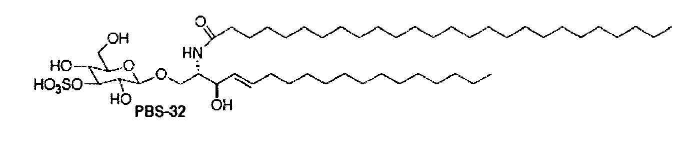

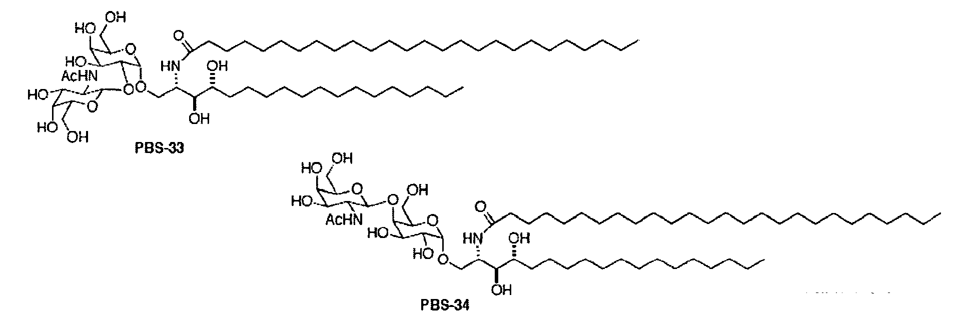

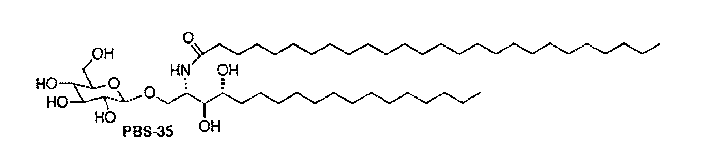

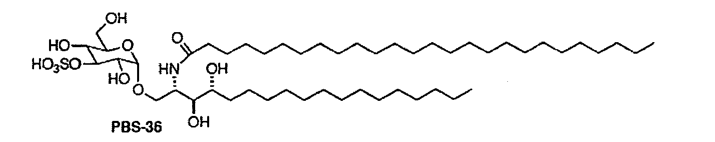

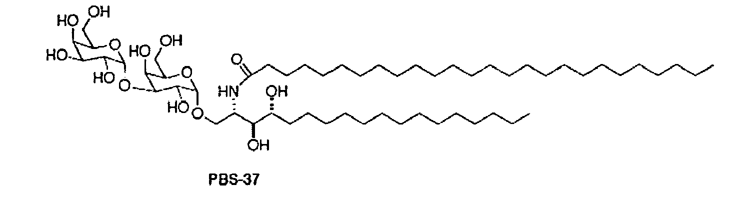

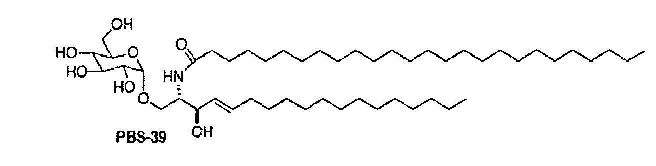

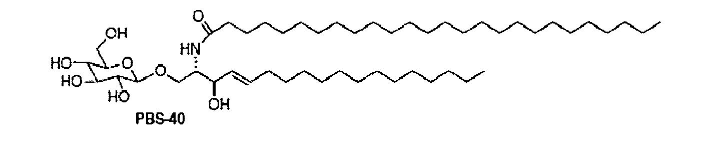

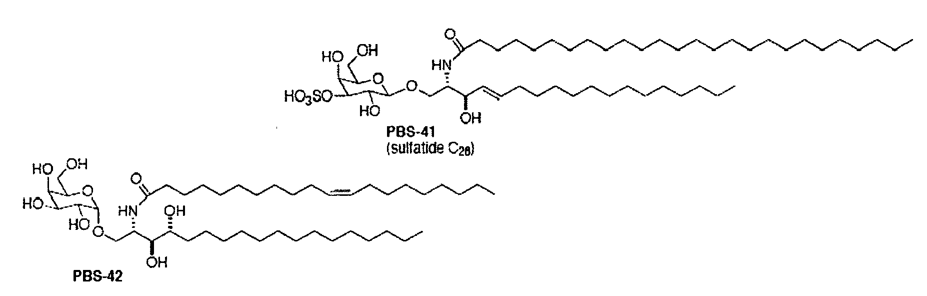

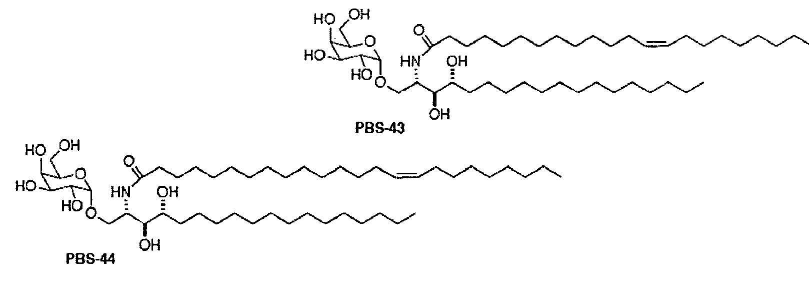

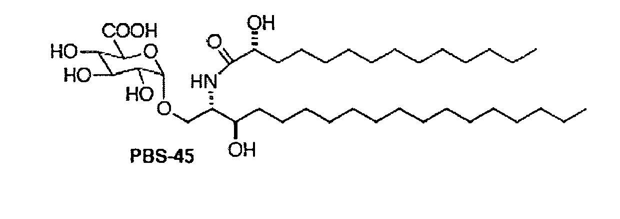

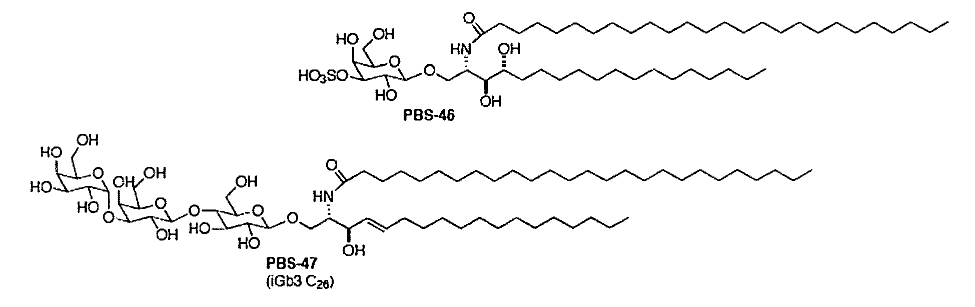

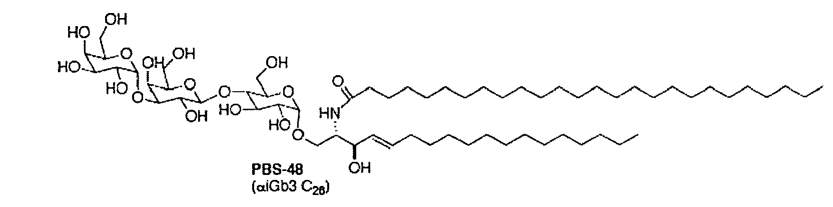

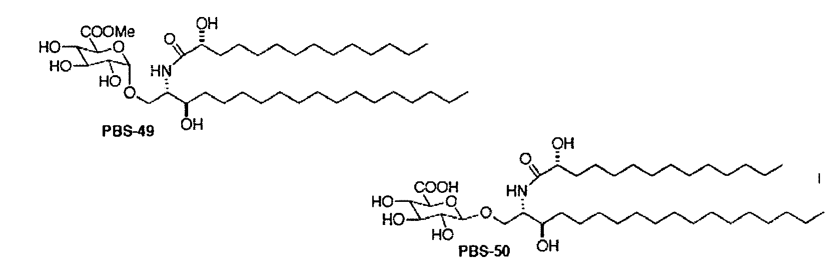

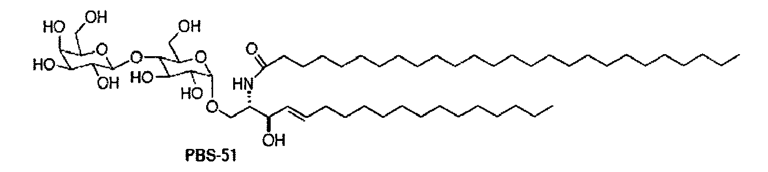

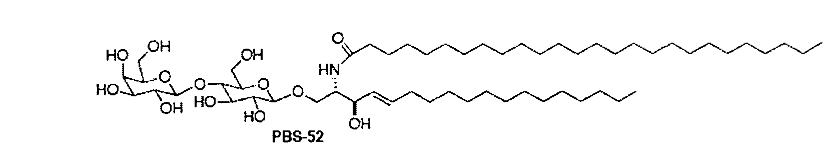

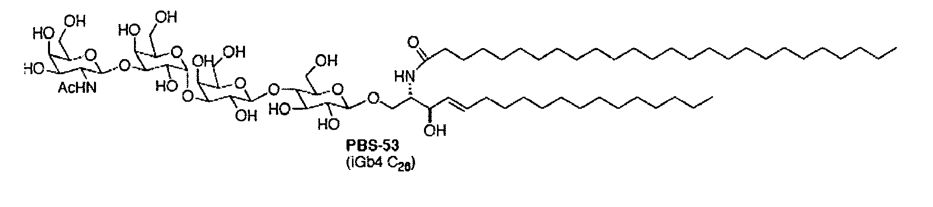

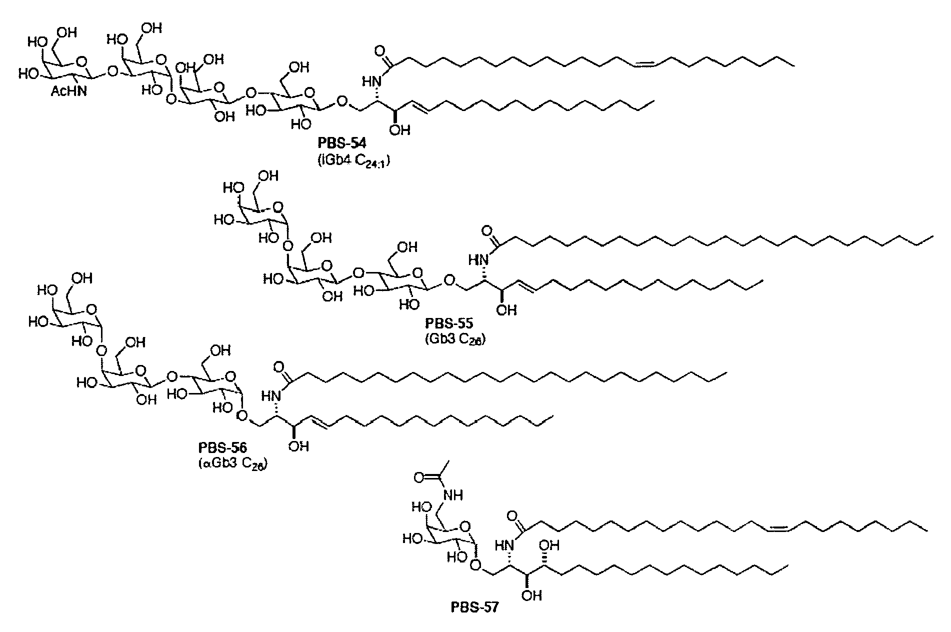

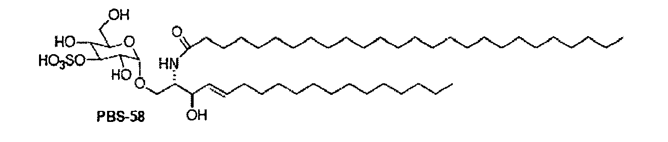

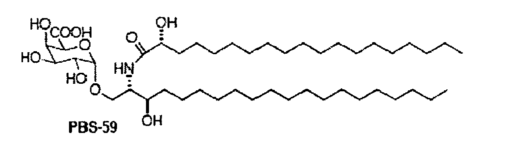

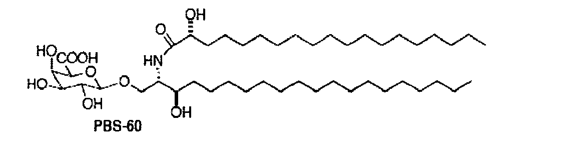

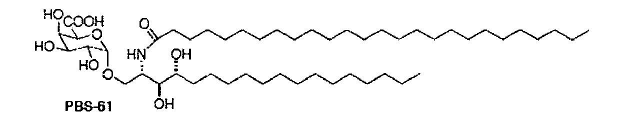

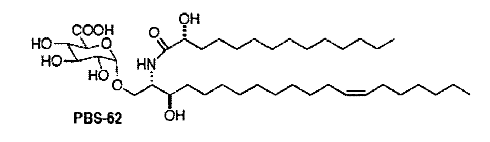

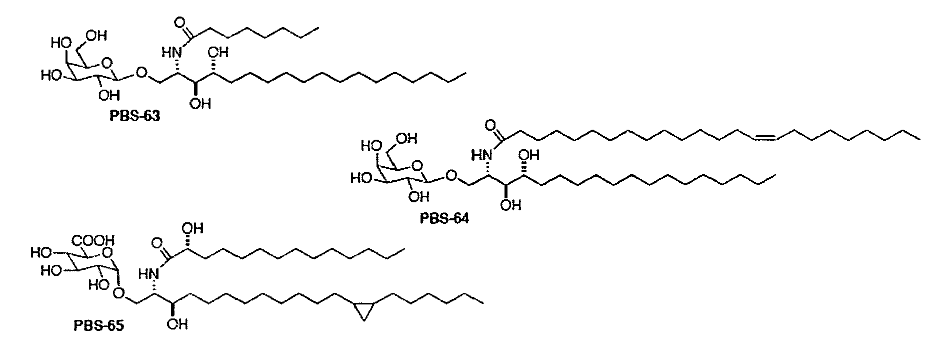

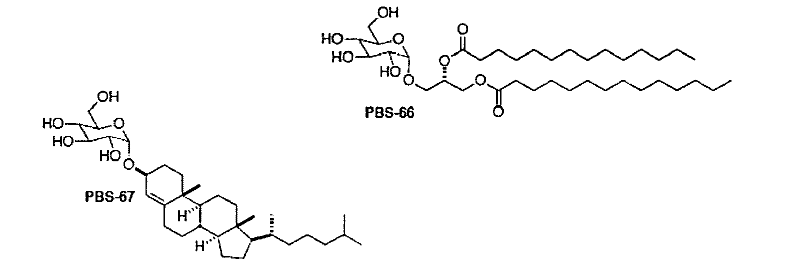

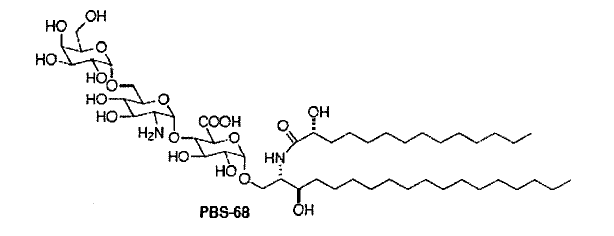

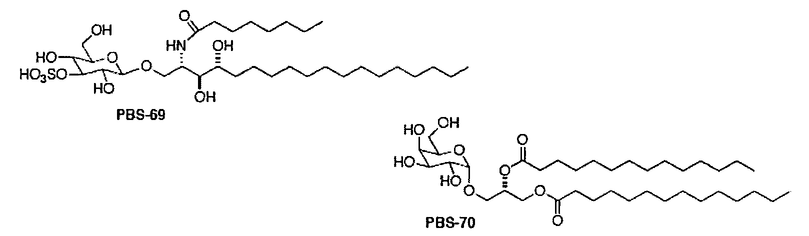

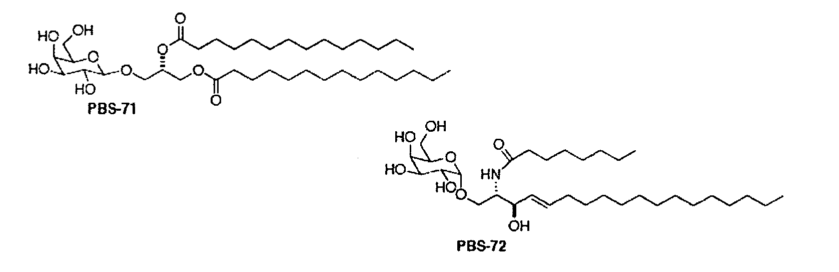

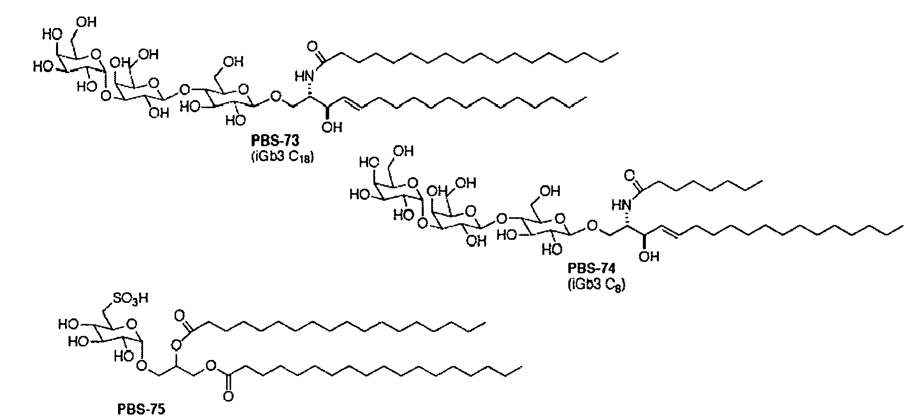

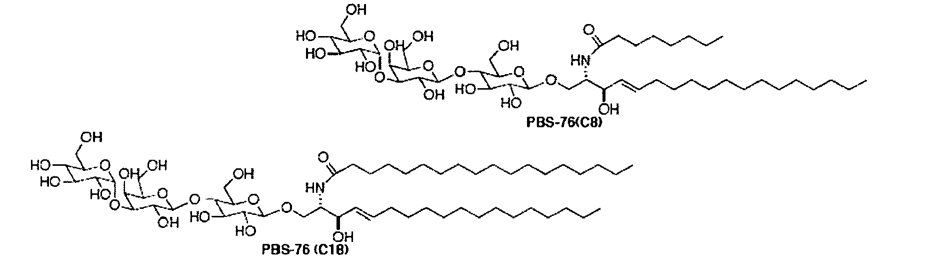

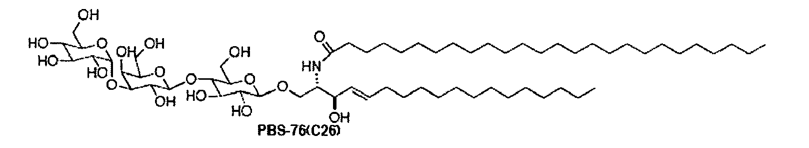

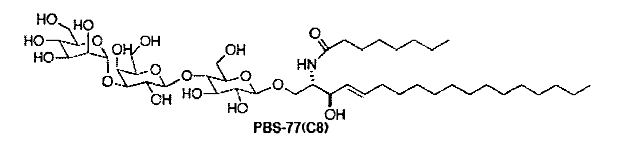

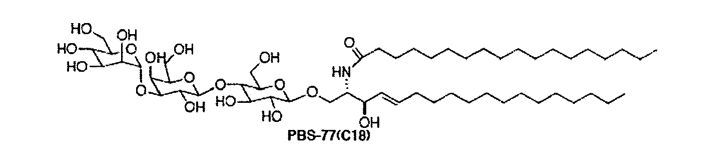

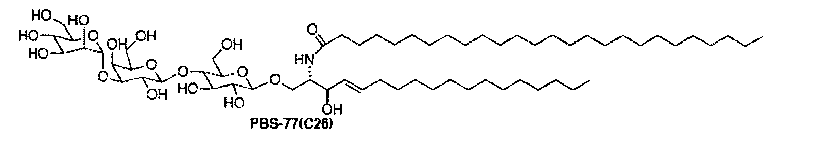

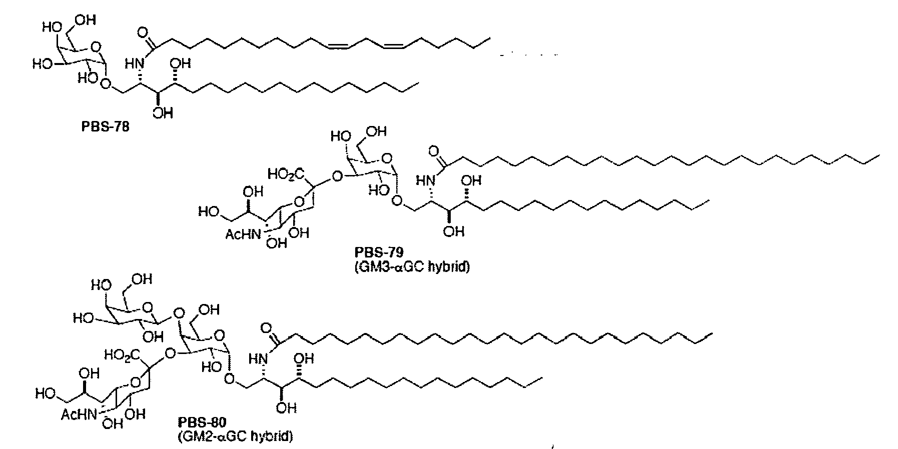

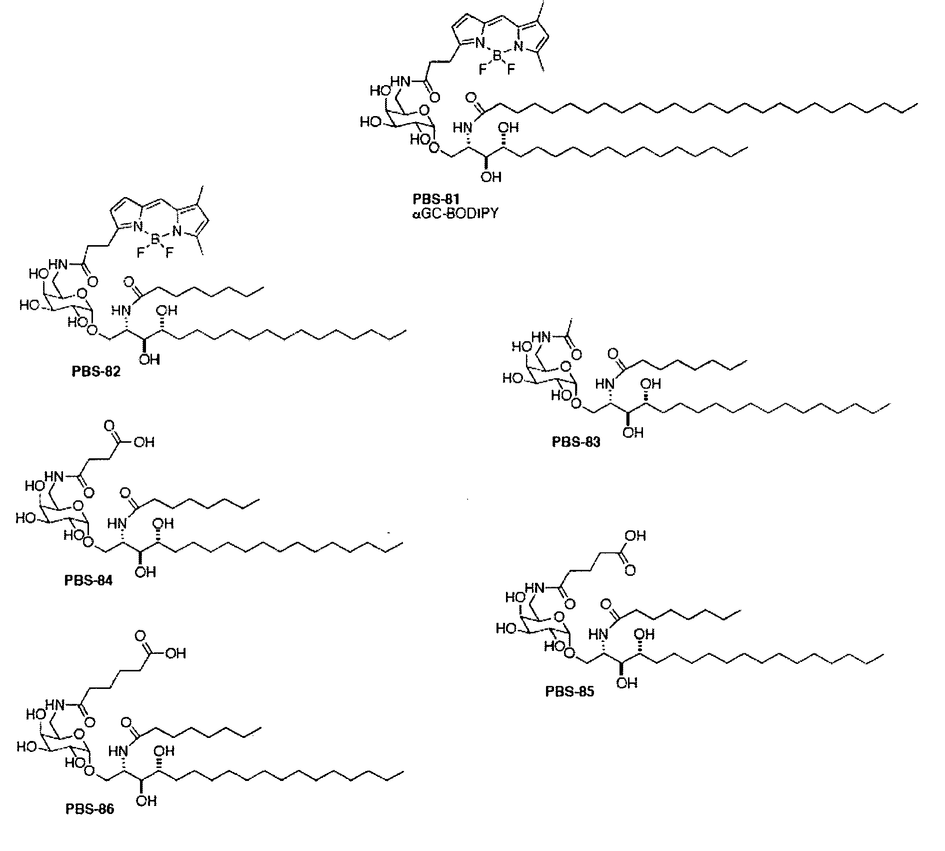

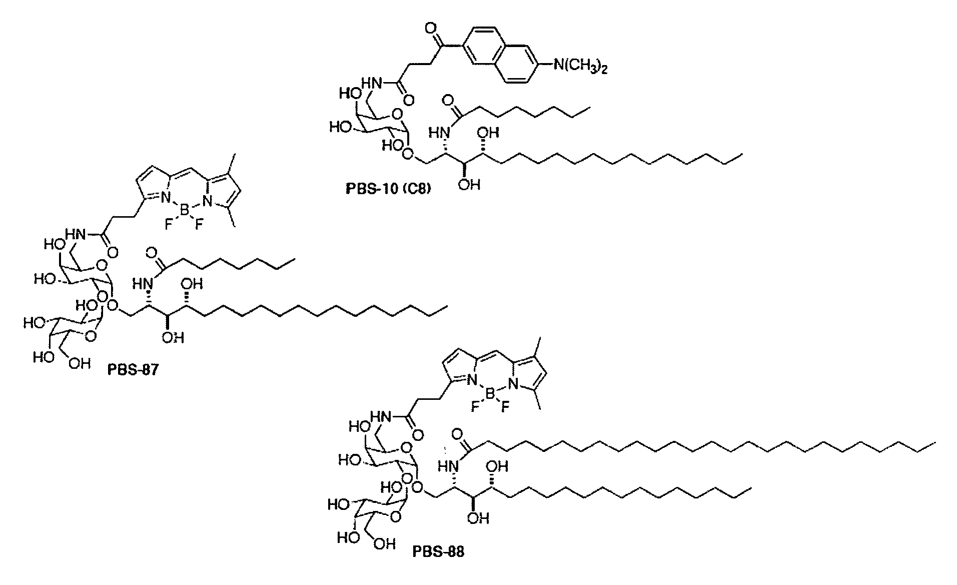

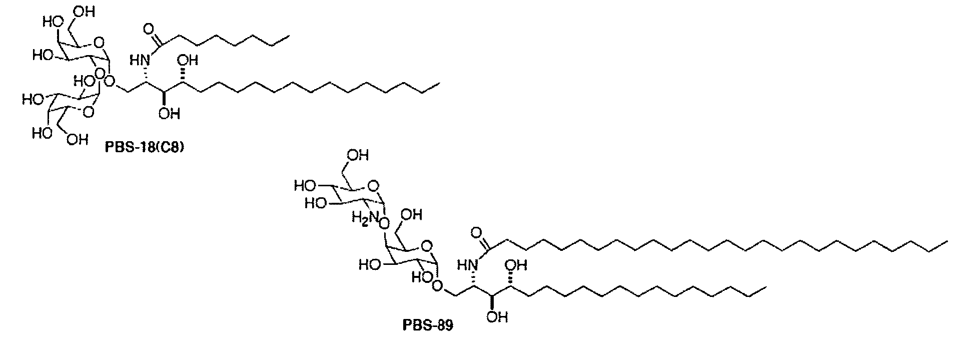

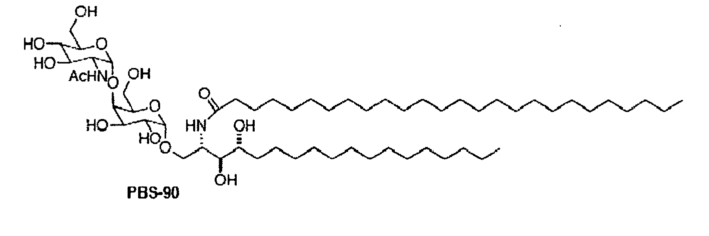

Bile salts are naturally occurring in the gastro-intestinal tractus (GIT). Derived from cholesterol, theY possess surfactant properties and take part to the lipid dispersion and solubilization processes during digestion. They are able to get into the cell membranes as well as to form mixed aggregates with phospholipids (lecithins) to give rise to so-called "mixed micelles".

According to a preferred embodiment, the derivative of galactosylceramides of the composition as defined above has the following formula (I): 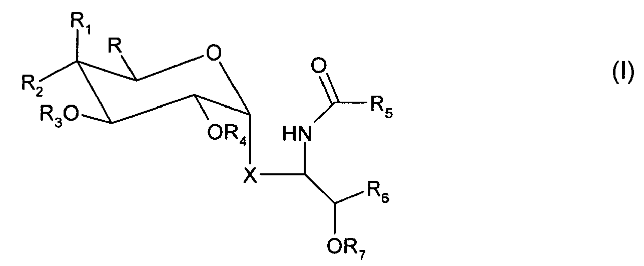 wherein:
- R₁ and R₂ represent H or OH, with the proviso that when R₁ represents H, then R₂ represents OH, and when R₁ represents OH, then R₂ represents H;
- R₃, R₄, and R₇ are each independently hydrogen, C₁-C₆ alkyl, C₆-C₁₂ aralkyl, or C₁-C₆ acyl;
- R represents a group chosen from the group consisting of:
   * -CH₂OH,
   * -CH₃,
   * -COOH, and
   * -CH₂-NHR₈,
      wherein:
      R₈ is chosen from the group consisting of:
         (i) hydrogen; or
         (ii) -SO₂R₁₀, wherein R₁₀ is:
            halo, hydroxyl, OR₁₁, OR₁₂, amino, NHR₁₁, N(R₁₁)₂, NHR₁₂, N(R₁₂)₂, aralkylamino, or
            C₁-C₁₂ alkyl, said alkyl group being optionally substituted with halo, hydroxy, oxo, nitro, OR₁₁, OR₁₂, acyloxy, amino, NHR₁₁, N(R₁₁)₂, NHR₁₂, N(R₁₂)₂, aralkylamino, mercapto, thioalkoxy, S(O)R₁₁, S(O)R₁₂, SO₂R₁₁, SO₂R₁₂, NHSO₂R₁₁, NHSO₂R₁₂, sulphate, phosphate, cyano, carboxyl, C(O)R₁₁, C(O)R₁₂, C(O)OR₁₁, C(O)NH₂, C(O)NHR₁₁, C(O)N(R₁₁)₂, C₃-C₁₀ cycloalkyl containing 0 to 3 R₁₃ group(s), C₃-C₁₀ heterocyclyl containing 0 to 3 R₁₃ group(s), C₂-C₆ alkenyl, C₂-C₆ alkynyl, C₅-C₁₀ cycloalkenyl, C₅-C₁₀ heterocycloalkenyl, C₆-C₂₀ aryl containing 0 to 3 R₁₄ group(s), or heteroaryl containing 0 to 3 R₁₄ group(s); or
            C₃-C₁₀ cycloalkyl, C₃-C₁₀ heterocyclyl, C₅-C₁₀ cycloalkenyl, or C₅-C₁₀ heterocycloalkenyl, said cycloalkyl, heterocyclyl, cycloalkenyl, or heterocycloalkenyl groups being optionally substituted with one or more halo, hydroxy, oxo, OR₁₁, OR₁₂, acyloxy, nitro, amino, NHR₁₁, N(R₁₁)₂, NR₁₂, N(R₁₂)₂, aralkylamino, mercapto, thioalkoxy, S(O)R₁₁, S(O)R₁₂, SO₂R₁₁, SO₂R₁₂, NHSO₂R₁₁, NHSO₂R₁₂, sulphate, phosphate, cyano, carboxyl, C(O)R₁₁, C(O)R₁₂, C(O)OR₁₁, C(O)NH₂, C(O)NHR₁₁, C(O)N(R₁₁)₂, alkyl, haloalkyl, C₃-C₁₀ cycloalkyl containing 0 to 3 R₁₃ group(s), C₃-C₁₀ heterocyclyl containing 0 to 3 R₁₃ group(s), C₂-C₆ alkenyl, C₂-C₆ alkynyl, C₅-C₁₀ cycloalkenyl, C₅-C₁₀ heterocycloalkenyl, C₆-C₂₀ aryl containing 0 to 3 R₁₄ group(s), or C₆-C₂₀ heteroaryl containing 0 to 3 R₁₄ group(s); or
            C₂-C₆ alkenyl, C₂-C₆ alkynyl, aryl, or heteroaryl, said alkenyl, alkynyl, aryl or heteroaryl groups being optionally substituted with one or more halo, hydroxy, OR₁₁, OR₁₂, acyloxy, nitro, amino, NHR₁₁, N(R₁₁)₂, NR₁₂, N(R₁₂)₂, aralkylamino, mercapto, thioalkoxy, S(O)R₁₁, S(O)R₁₂, SO₂R₁₁, SO₂R₁₂, NHSO₂R₁₁, NHSO₂R₁₂, sulphate, phosphate, cyano, carboxyl, C(O)R₁₁, C(O)R₁₂, C(O)OR₁₁, C(O)NH₂, C(O)NHR₁₁, C(O)N(R₁₁)₂, alkyl, haloalkyl, C₃-C₁₀ cycloalkyl containing 0 to 3 R₁₃ group(s), C₃-C₁₀ heterocyclyl containing 0 to 3 R₁₃ group(s), C₂-C₆ alkenyl, C₂-C₆ alkynyl, C₅-C₁₀ cycloalkenyl, C₅-C₁₀ heterocycloalkenyl, C₆-C₂₀ aryl containing 0 to 3 R₁₄ group(s), or C₆-C₂₀ heteroaryl containing 0 to 3 R₁₄ group(s); or
         (iii) -C(O)R₁₀, wherein R₁₀ is defined as above; or
         (iv) -C(R₁₀)₂(R₁₅), wherein R₁₀ is defined as above; R₁₅ is hydrogen, or has the same definition as R₁₀;
      R₁₁ being C₁-C₂₀ alkyl optionally substituted with halo, hydroxy, alkoxy, amino, alkylamino, dialkylamino, sulphate, or phosphate,
      R₁₂ being aryl optionally substituted with halo, haloalkyl, hydroxy, alkoxy, nitro, amino, alkylamino, dialkylamino, sulphate, or phosphate,
      R₁₃ being independently halo, haloalkyl, hydroxy, alkoxy, oxo, amino, alkylamino, dialkylamino, sulphate, or phosphate, and
      R₁₄ being independently halo, haloalkyl, hydroxy, alkoxy, nitro, amino, alkylamino, dialkylamino, sulphate, or phosphate,
- X represents a linkage chosen from the group consisting of: -CH₂-CH₂-,
- O-CH₂-, and -CH₂-CH=CH-;
- R₅ is chosen from the group consisting of:
   a) -(CH₂)ₓCH₃, x being an integer selected from 1 to 100,
   b) -(CH₂)_{x'}CH=CH(CH₂)_{y}CH₃, and
   c) -(CH₂)_{x'}CH=CH(CH₂)yCH=CH(CH₂)_{z}CH₃,
   x', y, and z being integers independently selected from 1 to 14;
- R₆ is chosen from the group consisting of:
   a) -CH(OR₁₆)(R₁₇), in particular a group having the following formula (A): 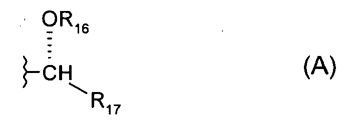
   b) -CH=CH-R₁₇, and
   c) -CH₂-CH₂-R₁₇,
      wherein:
      R₁₆ represents hydrogen, C₁-C₆ alkyl, C₆-C₁₂ aralkyl, or C₁-C₆ acyl;
      R₁₇ represents a linear or branched C₃-C₁₀₀ alkyl.

As used herein, the term "alkyl" refers to a hydrocarbon chain that may be a straight chain or branched chain, containing the indicated number of carbon atoms. For example, C₁-C₁₂ alkyl indicates that the group may have from 1 to 12 (inclusive) carbon atoms in it. The terms "arylalkyl" or "aralkyl" refer to an alkyl moiety in which an alkyl hydrogen atom is replaced by an aryl group. Examples of "arylalkyl" or "aralkyl" include benzyl and 9-fluorenyl groups.

The term "acyl" refers to an alkylcarbonyl, cycloalkylcarbonyl, arylcarbonyl, heterocyclylcarbonyl, or heteroarylcarbonyl substituent, any of which may be further substituted by substituents.

The term "cycloalkyl" as employed herein includes saturated cyclic, bicyclic, tricyclic, or polycyclic hydrocarbon groups having 3 to 12 carbons, wherein any ring atom capable of substitution can be substituted by a substituent. Examples of cycloalkyl moieties include, but are not limited to, cyclohexyl and adamantyl.

The term "aryl" refers to an aromatic monocyclic, bicyclic, or tricyclic hydrocarbon ring system, wherein any ring atom capable of substitution can be substituted by a substituent. Examples of aryl moieties include, but are not limited to, phenyl, naphthyl, and anthracenyl.

The term "heterocyclyl" refers to a nonaromatic 3-10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e. g. , carbon atoms and 1-3, 1-6, or1-9 heteroatoms of N, O, or S if monocyclic, bicyclic, or tricyclic, respectively), wherein any ring atom capable of substitution can be substituted by a substituent.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from 0, N, or S (e. g. , carbon atoms and 1-3, 1-6, or 1-9 heteroatoms of N, O, or S if monocyclic, bicyclic, or tricyclic, respectively), wherein any ring atom capable of substitution can be substituted by a substituent.

The term "oxo" refers to an oxygen atom, which forms a carbonyl when attached to carbon, an N-oxide when attached to nitrogen, and a sulfoxide or sulfone when attached to sulfur.

The term "substituents" refers to a group "substituted" on an alkyl, cycloalkyl, heterocyclyl, aryl, or heteroaryl group at any atom of that group. Suitable substituents include, without limitation, alkyl, alkenyl, alkynyl, alkoxy, halo, hydroxy, cyano, nitro, amino, SO₃H, sulfate, phosphate, perfluoroalkyl, perfluoroalkoxy, methylenedioxy, ethylenedioxy, carboxyl, oxo, thioxo, imino (alkyl, aryl, aralkyl), S(O)ₙ alkyl (where n is 0-2), S(O)ₙ aryl (where n is 0-2), S(O)ₙ heteroaryl (where n is 0-2), S(O)ₙ heterocyclyl (where n is 0- 2), amine (mono-, di-, alkyl, cycloalkyl, aralkyl, heteroaralkyl, and combinations thereof), ester (alkyl, aralkyl, heteroaralkyl), amide (mono-, di-, alkyl, aralkyl, heteroaralkyl, and combinations thereof), sulfonamide (mono-, di-, alkyl, aralkyl, heteroaralkyl, and combinations thereof), unsubstituted aryl, unsubstituted heteroaryl, unsubstituted heterocyclyl, and unsubstituted cycloalkyl.

The terms "alkylamino" and "dialkylamino" refer to -NH(alkyl) and -NH(alkyl)₂ radicals, respectively.

The term "aralkylamino" refers to a -NH(aralkyl) radical.

The term "alkoxy" refers to an -O-alkyl radical.

The term "mercapto" refers to an SH radical.

The term "thioalkoxy" refers to an -S-alkyl radical.

The term "cycloalkenyl" as employed herein includes partially unsaturated, nonaromatic, cyclic, bicyclic, tricyclic, or polycyclic hydrocarbon groups having 5 to 12 carbons, preferably 5 to 8 carbons, wherein any ring atom capable of substitution can be substituted by a substituent. Examples of cycloalkyl moieties include, but are not limited to cyclohexenyl, cyclohexadienyl, or norbornenyl.

The term "heterocycloalkenyl" refers to a partially saturated, nonaromatic 5-10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein any ring atom capable of substitution can be substituted by a substituent.

R₅ can have 1 to 100 methylene (CH₂) groups (i.e. R₅ is (CH₂)ₓCH₃ and x=1-100). In particular R₅ may have 1-75 CH₂ groups, 1-50 CH₂ groups, 1-25 CH₂ groups, 1-20 CH₂ groups, 1-15 CH₂ groups, 1-10 CH₂ groups or 1-5 CH₂ groups. Preferably, R₅ has 15-25 CH₂ groups. More preferably, R₆ has 20-25 CH₂ groups.

In certain embodiments R₅ contains 22 or 24 CH₂ groups (x=22 or x=24).

R₁₇ may be in particular a linear or branched C₃-C₇₅ alkyl, C₃-C₅₀ alkyl, C₃-C₂₅ alkyl, C₃-C₂₀ alkyl, C₃-C₁₅ alkyl, C₁₀-C₁₅ alkyl or C₃-C₁₀ alkyl.

In certain embodiments R₁₇ is a linear alkyl group of 14 carbon atoms.

Preferably R₅ is C₂₅H₅₁ and R₁₇ is C₁₄H₂₉. More preferably, R₅ is C₂₃H₄₅ and R₁₇ is C₁₄H₂₉. In another preferred embodiment, R₅ is C₂₃H₄₇ and R₁₇ is C₁₄H₂₉.

When R₁-R₄ are other than hydrogen, preferably each are independently methyl, benzyl or acetyl.

According to a preferred embodiment, the derivative of galactosylceramides has the following formula (I-1): 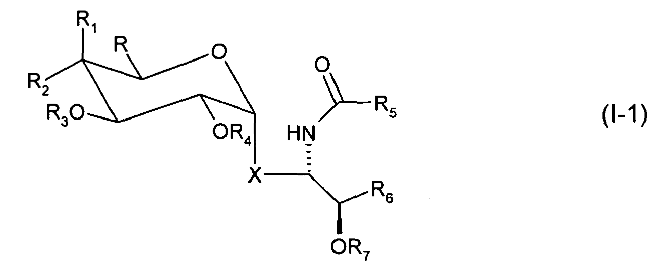 wherein R, R₁, R₂, R₃, R₄, R₅, R₆, R₇, and X are as defined above in formula (I).

According to an advantageous embodiment, the derivative of galactosylceramides has the formula (I) or (I-1), wherein R represents -CH₂-NHR₈, R₈ being as defined above in formula (I).

Thus, in such a case, the composition of the invention contains a derivative of galactosylceramide having one of the following formulae:

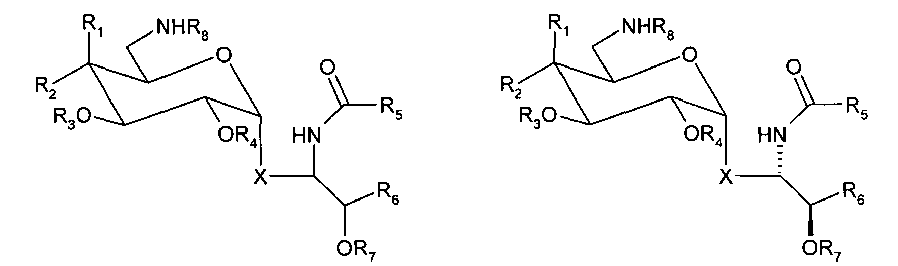

According to an advantageous embodiment, the derivative of galactosylceramides is a compound of formula (I-1) as mentioned above, wherein R is -CH₂-NH₂, R₁ is OH, R₂, R₃, R₄, and R₇ are hydrogen, X is -O-CH₂-, R₅ is C₂₅H₅₁, and R₆ is a group of formula (A) as defined in claim 2, wherein R₁₇ is C₁₄H₂₉.

According to another advantageous embodiment, the derivative of galactosylceramides is a compound of formula (I-1) as mentioned above, wherein R is -CH₂-NH-C(O)-CH₃, R₁ is OH, R₂, R₃, R₄, and R₇ are hydrogen, X is -O-CH₂-, R₅ is C₂₅H₅₁, and R₆ is a group of formula (A) as defined in claim 2, wherein R₁₇ is C₁₄H₂₉.

According to another advantageous embodiment, the derivative of galactosylceramides is a compound of formula (I-1) as mentioned above, wherein R is -CH₂-NH-C(O)-CH₃, R₁ is OH, R₂, R₃, R₄, and R₇ are hydrogen, X is -O-CH₂-, R₅ is C₂₃H₄₅, and R₆ is a group of formula (A) as defined in claim 2, wherein R₁₇ is C₁₄H₂₉.

According to another advantageous embodiment, the derivative of galactosylceramides is a compound of formula (I-1) as mentioned above, wherein R is -CH₂-NH-C(O)-CH₃, R₁ is OH, R₂, R₃, R₄, and R₇ are hydrogen, X is -O-CH₂-, R₅ is C₂₃H₄₇, and R₆ is a group of formula (A) as defined in claim 2, wherein R₁₇ is C₁₄H₂₉.

The present invention also relates to a composition as mentioned above, wherein the bile salt is sodium taurocholate or sodium deoxycholate.

The present invention also relates to a composition as mentioned above, characterized in that the molar ratio between the derivative of galactosylceramides, in particular of formula (I) or (I-1), and the bile salt, such as sodium taurocholate or sodium deoxycholate, is from 10:1 to 1:10, and is in particular 1:1.

The present invention also relates to a composition as mentioned above, further containing an organic solvent chosen in particular from ethanol and methanol.

The present invention also relates to a method for the preparation of a composition as mentioned above, comprising the co-solubilization of the derivative of galactosylceramides and the bile salt into a solvent, followed by the evaporation of said solvent.

### EXAMPLE

The derivative of galactosylceramides used as active ingredient is named PBS57 and has the following formula:

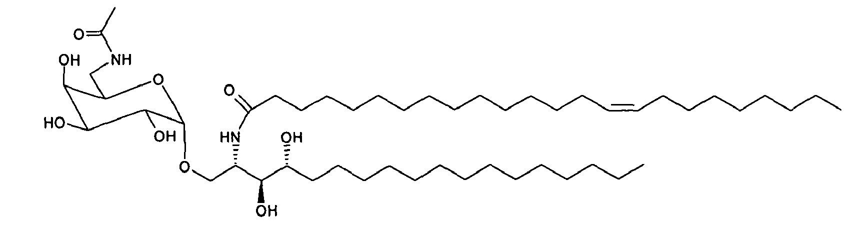

### Formation of mixed micelles with bile salts

PBS57 was solubilized by the use of bile salts, in order to give rise to mixed micellar solution (which are clear solutions) that could have many advantages on a pharmaceutical process point of view.

### 1- Preliminary testing of two bile salts

The mixed-micelle solution manufacturing process requires co-solubilization of the lipid and the bile salt into a solvent (ethanol) that is then evaporated before the final aqueous medium is added to obtain the mixed-micellar solution. A 5% glucose solution was used as the aqueous reconstitution medium here.

Both sodium taurocholate and deoxycholate were tested. A first indication that the mixed aggregates are truly formed is the clear aspect of the solution.

Results of the aspect after reconstitution by 5% glucose are presented in the following table:

| **Excipient** | **Active compound** | **Macroscopic observation right after reconstitution with 5% glucose** |
|---|---|---|
| Taurocholate | PBS57 | Slightly turbid |
| Deoxycholate | PBS57 | Turbid with large aggregates, flocculation slowly turning to sedimentation |

PBS57 concentration in the above 2 experiments was 50 µg/mL. The ratio bile salt / PBS57 was here 1/1.

### 2- "Micelle to vesicle" phase transition

It is well established that phospholipids/bile salt mixed aggregates are going through a phase transition to form vesicles (liposomes) once diluted.

The same experiment was carried out on a mixed micellar solution obtained from sodium taurocholate and PBS57 (50 µg/mL).

The clear solution rapidly turned into a slightly turbid suspension upon dilution, showing that the phase transition indeed occurred with PBS57 mixed aggregates, as it would have occurred with phospholipids/bile salt mixed micellar solution.

Particle size analysis illustrates very well this phase transition, as shown hereafter. The particle sizes are measured on a Malvern particle size analyzer (HPPS).

| | Main population particle size (nm) |
|---|---|
| taurocholate/PBS57 (1M/1M) | 12 |
| Dilution factor = 2 | 3624 |
| Dilution factor = 4 | 6348 |
| Dilution factor = 8 | 19942 |

It is proposed to take advantage of this phase transition to generate *in situ, in vivo,* following injection, PBS57 liposomes in an optimal physical state, so that the vaccine response would be maximized.

### 3- Manufacturing of a 200 µg/mL mixed-micellar solution of PBS57

Solutions of PBS57 at 200 µg/mL associated (equimolar ratio) to sodium taurocholate or sodium deoxycholate were manufactured by the same protocol. The resulting solution was perfectly clear after reconstitution.

## Claims

1. A composition comprising:
- at least one derivative of galactosylceramides, and
- at least one bile salt.

2. The composition of claim 1, wherein the derivative of galactosylceramides has the following formula (I): 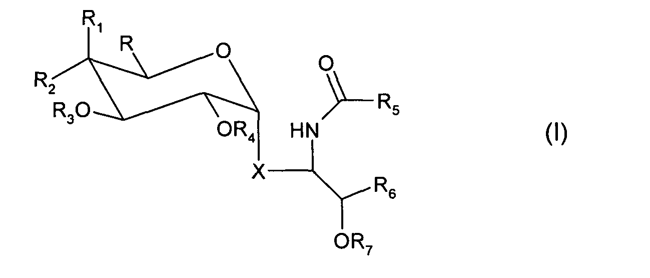 wherein:
- R₁ and R₂ represent H or OH, with the proviso that when R₁ represents H, then R₂ represents OH, and when R₁ represents OH, then R₂ represents H;
- R₃, R₄, and R₇ are each independently hydrogen, C₁-C₆ alkyl; C₆-C₁₂ aralkyl, or C₁-C₆ acyl;
- R represents a group chosen from the group consisting of:
* -CH₂OH,
* -CH₃,
* -COOH, and
* -CH₂-NHR₈,
wherein:
R₈ is chosen from the group consisting of:
(v) hydrogen; or
(vi) -SO₂R₁₀, wherein R₁₀ is:
halo, hydroxyl, OR₁₁, OR₁₂, amino, NHR₁₁, N(R₁₁)₂, NHR₁₂, N(R₁₂)₂, aralkylamino, or
C₁-C₁₂ alkyl, said alkyl group being optionally substituted with halo, hydroxy, oxo, nitro, OR₁₁, OR₁₂, acyloxy, amino, NHR₁₁, N(R₁₁)₂, NHR₁₂, N(R₁₂)₂, aralkylamino, mercapto, thioalkoxy, S(O)R₁₁, S(O)R₁₂, SO₂R₁₁, SO₂R₁₂, NHSO₂R₁₁, NHSO₂R₁₂, sulphate, phosphate, cyano, carboxyl, C(O)R₁₁, C(O)R₁₂, C(O)OR₁₁, C(O)NH₂, C(O)NHR₁₁, C(O)N(R₁₁)₂, C₃-C₁₀ cycloalkyl containing 0 to 3 R₁₃ group(s), C₃-C₁₀ heterocyclyl containing 0 to 3 R₁₃ group(s), C₂-C₆ alkenyl, C₂-C₆ alkynyl, C₅-C₁₀ cycloalkenyl, C₅-C₁₀ heterocycloalkenyl, C₆-C₂₀ aryl containing 0 to 3 R₁₄ group(s), or heteroaryl containing 0 to 3 R₁₄ group(s); or
C₃-C₁₀ cycloalkyl, C₃-C₁₀ heterocyclyl, C₅-C₁₀ cycloalkenyl, or C₅-C₁₀ heterocycloalkenyl, said cycloalkyl, heterocyclyl, cycloalkenyl, or heterocycloalkenyl groups being optionally substituted with one or more halo, hydroxy, oxo, OR₁₁, OR₁₂, acyloxy, nitro, amino, NHR₁₁, N(R₁₁)₂, NR₁₂, N(R₁₂)₂, aralkylamino, mercapto, thioalkoxy, S(O)R₁₁, S(O)R₁₂, SO₂R₁₁, SO₂R₁₂, NHSO₂R₁₁, NHSO₂R₁₂, sulphate, phosphate, cyano, carboxyl, C(O)R₁₁, C(O)R₁₂, C(O)OR₁₁, C(O)NH₂, C(O)NHR₁₁, C(O)N(R₁₁)₂, alkyl, haloalkyl, C₃-C₁₀ cycloalkyl containing 0 to 3 R₁₃ group(s), C₃-C₁₀ heterocyclyl containing 0 to 3 R₁₃ group(s), C₂-C₆ alkenyl, C₂-C₆ alkynyl, C₅-C₁₀ cycloalkenyl, C₅-C₁₀ heterocycloalkenyl, C₆-C₂₀ aryl containing 0 to 3 R₁₄ group(s), or C₆-C₂₀ heteroaryl containing 0 to 3 R₁₄ group(s); or
C₂-C₆ alkenyl, C₂-C₆ alkynyl, aryl, or heteroaryl, said alkenyl, alkynyl, aryl or heteroaryl groups being optionally substituted with one or more halo, hydroxy, OR₁₁, OR₁₂, acyloxy, nitro, amino, NHR₁₁, N(R₁₁)₂, NR₁₂, N(R₁₂)₂, aralkylamino, mercapto, thioalkoxy, S(O)R₁₁, S(O)R₁₂, SO₂R₁₁, SO₂R₁₂, NHSO₂R₁₁, NHSO₂R₁₂, sulphate, phosphate, cyano, carboxyl, C(O)R₁₁, C(O)R₁₂, C(O)OR₁₁, C(O)NH₂, C(O)NHR₁₁, C(O)N(R₁₁)₂, alkyl, haloalkyl, C₃-C₁₀ cycloalkyl containing 0 to 3 R₁₃ group(s), C₃-C₁₀ heterocyclyl containing 0 to 3 R₁₃ group(s), C₂-C₆ alkenyl, C₂-C₆ alkynyl, C₅-C₁₀ cycloalkenyl, C₅-C₁₀ heterocycloalkenyl, C₆-C₂₀ aryl containing 0 to 3 R₁₄ group(s), or C₆-C₂₀ heteroaryl containing 0 to 3 R₁₄ group(s); or
(vii) -C(O)R₁₀, wherein R₁₀ is defined as above; or
(viii) -C(R₁₀)2(R₁₅), wherein R₁₀ is defined as above; R₁₅ is hydrogen, or has the same definition as R₁₀;
R₁₁ being C₁-C₂₀ alkyl optionally substituted with halo, hydroxy, alkoxy, amino, alkylamino, dialkylamino, sulphate, or phosphate,
R₁₂ being aryl optionally substituted with halo, haloalkyl, hydroxy, alkoxy, nitro, amino, alkylamino, dialkylamino, sulphate, or phosphate,
R₁₃ being independently halo, haloalkyl, hydroxy, alkoxy, oxo, amino, alkylamino, dialkylamino, sulphate, or phosphate, and
R₁₄ being independently halo, haloalkyl, hydroxy, alkoxy, nitro, amino, alkylamino, dialkylamino, sulphate, or phosphate,
- X represents a linkage chosen from the group consisting of: -CH₂-CH₂-,
- O-CH₂-, and -CH₂-CH=CH-;
- R₅ is chosen from the group consisting of:
a) -(CH₂)ₓCH₃, x being an integer selected from 1 to 100,
b) -(CH₂)_{x'}CH=CH(CH₂)_{y}CH₃, and
c) -(CH₂)_{x'}CH=CH(CH₂)_{y}CH=CH(CH₂)_{z}CH₃,
x', y, and z being integers independently selected from 1 to 14;
- R₆ is chosen from the group consisting of:
a) -CH(OR₁₆)(R₁₇), in particular a group having the following formula (A): 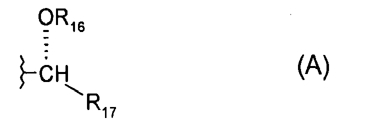
b) -CH=CH-R₁₇, and
c) -CH₂-CH₂-R₁₇,
wherein:
R₁₆ represents hydrogen, C₁-C₆ alkyl, C₆-C₁₂ aralkyl, or C₁-C₆ acyl;
R₁₇ represents a linear or branched C₃-C₁₀₀ alkyl.

3. The composition of claim 1 or 2, wherein the derivative of galactosylceramides has the following formula (I): 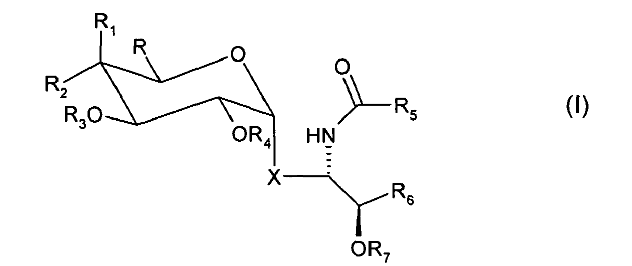 wherein R, R₁, R₂, R₃, R₄, R₅, R₆, R₇, and X are as defined in claim 2.

4. The composition of any of claims 1 to 3, wherein R represents -CH₂-NHR₈, R₈ being as defined in claim 2.

5. The composition of claim 3, wherein R is -CH₂-NH₂, R₁ is OH, R₂, R₃, R₄, and R₇ are hydrogen, X is -O-CH₂-, R₅ is C₂₅H₅₁, and R₆ is a group of formula (A) as defined in claim 2, wherein R₁₇ is C₁₄H₂₉.

6. The composition of claim 3, wherein R is -CH₂-NH-C(O)-CH₃, R₁ is OH, R₂, R₃, R₄, and R₇ are hydrogen, X is -O-CH₂-, R₅ is C₂₅H₅₁, and R₆ is a group of formula (A) as defined in claim 2, wherein R₁₇ is C₁₄H₂₉.

7. The composition of claim 3, wherein R is -CH₂-NH-C(O)-CH₃, R₁ is OH, R₂, R₃, R₄, and R₇ are hydrogen, X is -O-CH₂-, R₅ is C₂₃H₄₅, and R₆ is a group of formula (A) as defined in claim 2, wherein R₁₇ is C₁₄H₂₉.

8. The composition of claim 3, wherein R is -CH₂-NH-C(O)-CH₃, R₁ is OH, R₂, R₃, R₄, and R₇ are hydrogen, X is -O-CH₂-, R₅ is C₂₃H₄₇, and R₆ is a group of formula (A) as defined in claim 2, wherein R₁₇ is C₁₄H₂₉.

9. The composition of any of claims 1 to 8, wherein the bile salt is sodium taurocholate or sodium deoxycholate.

10. The composition of any of claims 1 to 9, **characterized in that** the molar ratio between the derivative of galactosylceramides and the bile salt is from 10:1 to 1:10, and is in particular 1:1.

11. The composition of any of claims 1 to 10, **characterized in that** it further contains an organic solvent chosen in particular from ethanol and methanol.

12. A method for the preparation of a composition of any of claims 1 to 11, comprising the co-solubilization of the derivative of galactosylceramides and the bile salt into a solvent, followed by the evaporation of said solvent.
